(19) Europäisches Patentamt
European Patent Office
Office européen des brevets

(11) Publication number: 0 054 974
B1

(12) **EUROPEAN PATENT SPECIFICATION**

(45) Date of publication of patent specification: 10.07.85

(21) Application number: 81110748.1

(22) Date of filing: 23.12.81

(51) Int. Cl.⁴: C 07 D 233/60, C 07 D 233/61, A 01 N 43/50, C 07 D 401/12, C 07 D 405/12, A 61 K 31/415

# (54) Imidazolylpropanol compounds and their acid addition salts, and production and use thereof.

(30) Priority: 24.12.80 JP 184517/80
24.02.81 JP 26261/81
11.03.81 JP 35620/81

(43) Date of publication of application:
30.06.82 Bulletin 82/26

(45) Publication of the grant of the patent:
10.07.85 Bulletin 85/28

(84) Designated Contracting States:
AT BE CH DE FR GB IT LI NL SE

(56) References cited:
EP-A-0 040 345
EP-A-0 043 419
DE-A-1 954 706
DE-A-2 820 489
FR-A-2 081 542
FR-A-2 299 807
FR-A-2 398 056
US-A-4 036 974
US-A-4 215 220

**The file contains technical information submitted after the application was filed and not included in this specification**

(73) Proprietor: SUMITOMO CHEMICAL COMPANY, LIMITED
15 Kitahama 5-chome Higashi-ku
Osaka-shi Osaka 541 (JP)

(72) Inventor: Saji, Ikutaro
8-0-401, Aobaokaminami
Suita Osaka (JP)
Inventor: Tamoto, Katsumi
1-8-1-403, Natsumidai
Funabashi Chiba (JP)
Inventor: Aono, Shunji
3-15-33-303, Minamisakurazuka
Toyonaka Osaka (JP)
Inventor: Ichise, Katsuaki
2-2-30-310, Nagaoka
Nagaokakyo Kyoto (JP)
Inventor: Okuda, Takao
2-10-3-337, Sonehigashi-machi
Toyonaka Osaka (JP)
Inventor: Agui, Hideo
C1, 385-Canterbury Drive
Fredericton New Brunswick E3B 4M3 (CA)

(74) Representative: Vossius Vossius Tauchner Heunemann Rauh
Siebertstrasse 4 P.O. Box 86 07 67
D-8000 München 86 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

EP 0 054 974 B1

Courier Press, Leamington Spa, England.

**Description**

The present invention relates to imidazolylpropanol compounds and their acid addition salts, and production and use thereof.

The said imidazolylpropanol compounds are representable by the formula:

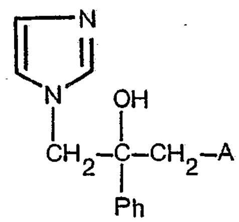

(I)

wherein

Ph is a phenyl group or a phenyl group substituted with halogen; and

A is —S—$R^1$ (wherein $R^1$ is a $C_1$—$C_{15}$ or a $C_{18}$ alkyl group, a phenyl group, a phenyl group substituted with at least one of $C_1$—$C_8$ alkyl, $C_1$—$C_8$ alkoxy and halogen, a benzyl group or a pyridyl group),

—O—$R^2$ (wherein $R^2$ is a hydrogen atom, a $C_1$—$C_{15}$ alkyl group, a $C_1$—$C_8$ alkyl group substituted with at least one of phenylthio and $C_1$—$C_8$ alkylthio, a $C_3$—$C_8$ alkenyl group, a $C_7$—$C_8$ aralkyl group, a $C_7$—$C_8$ aralkyl group substituted with halogen, or

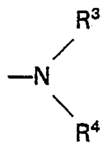

(wherein $R^3$ and $R^4$ are each a hydrogen atom, a $C_1$—$C_8$ alkyl group, a $C_3$—$C_6$ cycloalkyl group, a benzyl group, a benzyl group substituted with halogen, a $C_1$—$C_8$ alkyl group substituted with at least one of hydroxyl, morpholin and $C_1$—$C_8$ alkoxy, a $C_3$—$C_6$ alkynyl group, a furfuryl group or a tetrahydrofurfuryl group, or when taken together with the adjacent nitrogen atom, they represent a morpholino group, a piperazino group, an N-($C_1$—$C_8$)alkylpiperazino group, an N-phenylpiperazino group, an N-benzylpiperazino group, a pyrrolidino group or a piperidino group).

In the above definitions the term "$C_1$—$C_{15}$ alkyl" is intended to mean a straight or branched alkyl group having 1 to 15 carbon atoms (e.g. methyl, ethyl, n-propyl, isopropyl, n-butyl, isobutyl, tert-butyl, n-pentyl, n-hexyl, n-heptyl, n-octyl, n-nonyl, n-undecyl, n-dodecyl, n-tridecyl, n-tetradecyl, n-pentadecyl). Likewise, the term "$C_1$—$C_8$ alkyl" is intended to mean a straight or branched alkyl group having 1 to 8 carbon atoms (e.g. methyl, ethyl, n-propyl, isopropyl, n-butyl, isobutyl, tert-butyl, n-pentyl, n-hexyl, n-heptyl, n-octyl). Those having 1 to 4 carbon atoms are particularly preferred. The term "$C_7$—$C_8$ aralkyl" may be benzyl, phenethyl, etc. The term "$C_3$—$C_8$ alkenyl" includes allyl, 3-butenyl, 4-pentenyl, 5-hexenyl, etc. Those of $C_3$ to $C_6$ are especially preferred. The term "$C_3$—$C_6$ alkynyl" may be, for instance, propargyl, 3-butynyl, 4-pentynyl or 5-hexynyl. The term "$C_3$—$C_6$ cycloalkyl" may be cyclopropyl, cyclopentyl, cyclohexyl, etc. The term "halogen" covers fluorine, chlorine, bromine and iodine. When a phenyl group or an aralkyl group is substituted, not more than three substituents may be present on the benzene ring.

Among various compounds represented by the formula (I), those are preferred wherein Ph is a phenyl group substituted with halogen and A is a $C_1$—$C_{15}$ alkylthio group. Particularly preferred are those wherein Ph is a 2,4-dichlorophenyl group and A is a $C_1$—$C_{15}$ alkylthio group.

For preparation of the imidazolylpropanol compounds (I), there may be adopted various procedures, among which typical ones are as follows:

*Procedure A*

An epoxide compound of the formula:

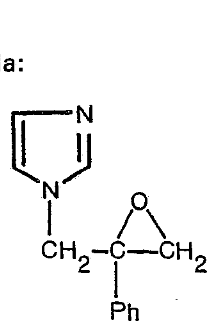

(II)

wherein Ph is as defined above is reacted with a reagent of either one of the formulas:

$\overset{\oplus}{M}\overset{\ominus}{S}-R^1$ (IIIa)

$\overset{\oplus}{M}\overset{\ominus}{O}-R^2$ (IIIb)

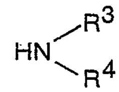

(IIIc)

wherein M is an alkali metal atom and $R^1$, $R^2$, $R^3$ and $R^4$ are each as defined above, when desired, in a liquid medium to give the imidazolylpropanol compound (I).

The amount of the reagent (IIIa), (IIIb) or (IIIc) may be usually from 1 to 3 molar equivalents to the epoxide compound (II). Even when a larger amount is used, no unfavorable influence on the reaction will be caused.

As the liquid medium, there may be used an aliphatic hydrocarbon (e.g. hexane), an aromatic hydrocarbon (e.g. benzene, toluene, xylene), an ether (e.g. dioxane, tetrahydrofuran), an alcohol (e.g. methanol, ethanol), acetonitrile, dimethylformamide, dimethylsulfoxide, acetone, water, etc. When the corresponding mercapto or hydroxy compound of the reagent (IIIa) and (IIIb) or the reagent (IIIc) is a liquid, it may be used in an excess amount so as to play the role of the liquid medium. The reaction may be effected usually at a temperature of from 0°C to the refluxing temperature of the reaction mixture, preferably of from room temperature to the said refluxing temperature.

The starting epoxide compound (II) may be prepared, for instance, by reacting a ketone compound of the formula:

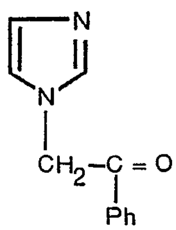

wherein Ph is as defined above with dimethyloxosulfonium methylide ($(CH_3)_2\overset{+}{S}O\overset{-}{C}H_2$) in a solvent such as dimethylsulfoxide at a temperature of 0 to 100°C (J.Am.Chem.Soc., *87,* page 1353 (1965)). The said ketone compound is per se known or can be produced by a per se conventional procedure (Japanese Patent Publication (examined) No. 39665/1975).

The reagent (IIIa) or (IIIb) may be prepared, for instance, by reacting the corresponding mercapto or hydroxyl compound with an equimolar amount of an alkali metal (e.g. metallic potassium, metallic lithium), an alkali metal hydride (e.g. sodium hydride) or an alkali metal hydroxide (e.g. sodium hydroxide, potassium hydroxide, lithium hydroxide), if desired, in a liquid medium. The thus prepared reagent (IIIa) or (IIIb) may be such as used in the reaction with the epoxide compound (II).

*Procedure B*

An epoxide compound of the formula:

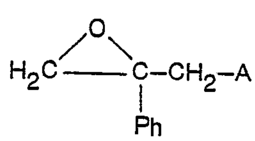

(IV)

wherein Ph and A are each as defined above is reacted with an imidazole compound of the formula:

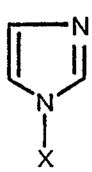

(V)

wherein X is potassium or sodium, when desired, in a liquid medium to give the imidazolylpropanol compound (I).

The amount of the imidazole compound (V) may be from 1 to 4 molar equivalents to the epoxide compound (IV). As the liquid medium, there may be used an aliphatic hydrocarbon (e.g. hexane), an aromatic hydrocarbon (e.g. benzene, toluene, xylene), an ether (dioxane, tetrahydrofuran), acetonitrile, acetone, etc. The reaction is usually effected at a temperature of from 0°C to the refluxing temperature of the reaction mixture, preferably of from room temperature to the refluxing temperature of the reaction mixture.

The starting epoxide compound (IV) may be produced by reacting a ketone compound of the formula:

$$\begin{array}{l} O{=}C{-}CH_2{-}A \\ \quad\ | \\ \quad Ph \end{array}$$

wherein Ph and A are each as defined above with dimethyloxosulfonium methylide ($(CH_3)_2\overset{+}{S}O\overset{-}{C}H_2$) in a solvent such as dimethylsulfoxide at a temperature of 0 to 100°C (J.Am.Chem.Soc., *87*, page 1353 (1965)). The said ketone compound is per se known or can be produced by a per se conventional procedure (Ber., *15*, page 2497 (1882)).

The imidazole compound (V) may be prepared by dissolving imidazole in an inert solvent and adding thereto an equimolar amount of an alkali metal hydride (e.g. sodium hydride). The produced imidazole compound (V) may be subjected as such to the reaction with the epoxide compound (IV).

The thus produced imidazolylpropanol compound (I) may be converted into its acid addition salt by treatment with an acid (e.g. hydrochloric acid, hydrobromic acid, phosphoric acid, nitric acid, acetic acid, maleic acid, succinic acid, fumaric acid, tartaric acid, citric acid, salicyclic acid, sorbic acid, lactic acid, oxalic acid).

The imidazolylpropanol compounds (I) exhibit a remarkable antimicrobial activity against various microorganisms, particularly fungi. Also, some of them are useful as agricultural fungicides.

The antifungal activity of some representatives of the imidazolylpropanol compounds (I) in in vitro test is as shown below.

TABLE 1

Antifungal activity in vitro

| Imidazolylpropanol Compound (I) 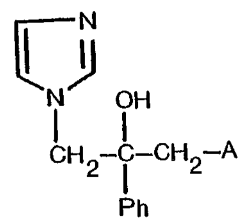 | | | Minimum inhibitory concentration (μg/ml) *1) | | |
|---|---|---|---|---|---|
| Ph | A | Salt | Candida albicans Ohta | Candida albicans MTU12001 | Trichophyton rubrum |
| 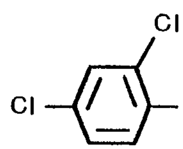 | 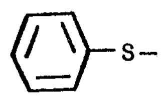 | Nitrate | 5 | 0.15 | 10 |
| 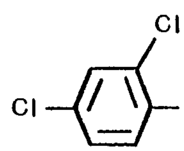 | 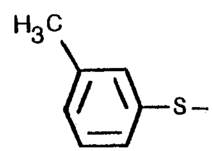 | Free | 0.6 | 0.15 | 5 |
| 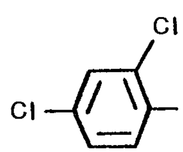 | 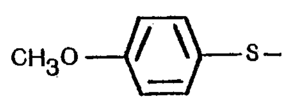 | Free | 5 | 0.3 | 20 |

TABLE 1 (Continued)

| Imidazolylpropanol Compound (I) 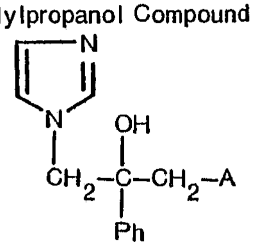 | | | Minimum inhibitory concentration ($\mu g/ml$) *1) | | |
|---|---|---|---|---|---|
| Ph | A | Salt | Candida albicans Ohta | Candida albicans MTU12001 | Trichophyton rubrum |
| 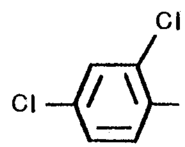 | 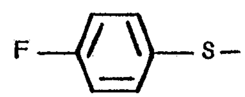 | Free | 2.5 | 0.3 | 40 |
| 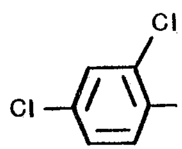 | 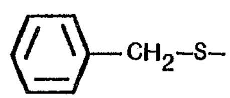 | Free | 2.5 | 0.075 | 5 |
| 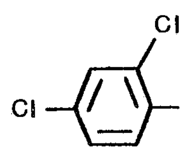 | $CH_3-(CH_2)_3-S-$ | Free | 1.25 | 0.075 | 0.6 |
| 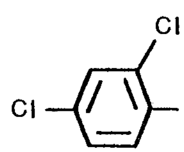 | 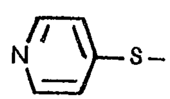 | Nitrate | 40 – 100 | 10 | 20 |

TABLE 1 (Continued)

| Imidazolylpropanol Compound (I) 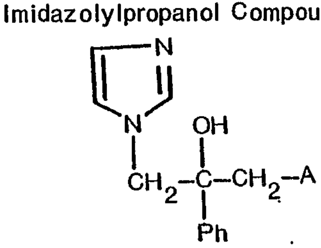 | | | Minimum inhibitory concentration ($\mu g/ml$) *1) | | |
|---|---|---|---|---|---|
| Ph | A | Salt | Candida albicans Ohta | Candida albicans MTU12001 | Trichophyton rubrum |
| 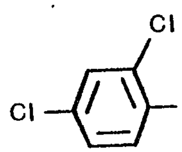 | 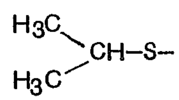 | Free | 40 | 0.6 | 5 |
| 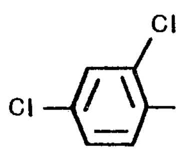 | $CH_3-(CH_2)_4-S-$ | Free | 5 | 1.25 | 1.25 |
| 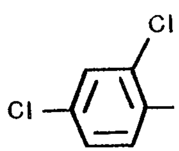 | $CH_3-(CH_2)_5-S-$ | Free | 2.5 | 0.3 | 0.6 |
| 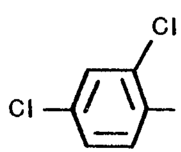 | $CH_3-(CH_2)_7-S-$ | Free | 1.25 | 0.15 | 0.3 |

TABLE 1 (Continued)

| Imidazolylpropanol Compound (I) 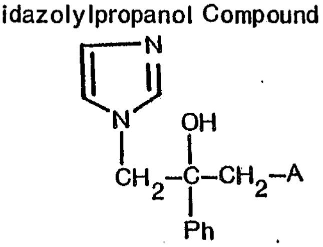 | | | Minimum inhibitory concentration (μg/ml) *1) | | |
|---|---|---|---|---|---|
| Ph | A | Salt | Candida albicans Ohta | Candida albicans MTU12001 | Trichophyton rubrum |
| 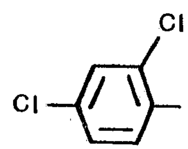 | $CH_3-(CH_2)_6-S-$ | Oxalate | 5 | 1.25 | 0.3 |
| 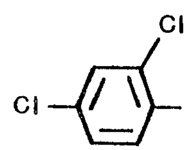 | $CH_3-(CH_2)_3-O-$ | Free | 40 | 5 | 0.6 |
| 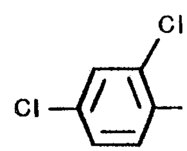 | 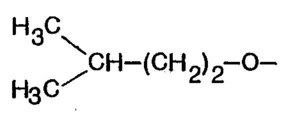 | Oxalate | 40 | 0.6 | 0.5 |
| 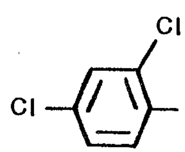 | $CH_2=CH-CH_2-O-$ | Oxalate | 40 – 100 | 1.25 | 10 |

TABLE 1 (Continued)

| Imidazolylpropanol Compound (i) 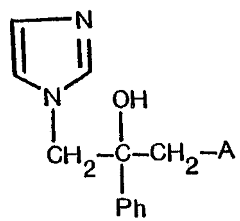 | | | Minimum inhibitory concentration ($\mu g/ml$) *1) | | |
|---|---|---|---|---|---|
| Ph | A | Salt | Candida albicans Ohta | Candida albicans MTU12001 | Trichophyton rubrum |
| 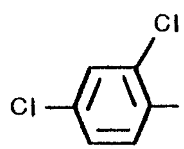 | 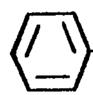 $-CH_2-O-$ | Oxalate | 40 | 10 | 0.6 |
| 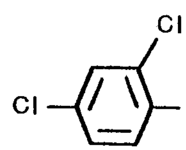 | 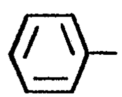 $-CH_2CH_2-O-$ | Oxalate | 40 | 0.6 | 2.5 |
| 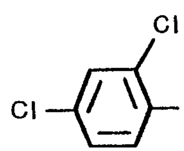 | 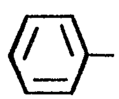 $-S-CH_2-CH_2-O-$ | Oxalate | 20 | 1.25 | 5 |
| 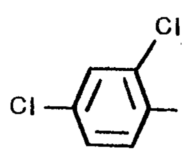 | $CH_3-(CH_2)_5-NH-$ | Free | 40 – 100 | 40 – 100 | 40 – 100 |

TABLE 1 (Continued)

| Imidazolylpropanol Compound (I) 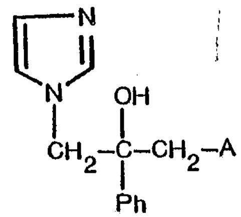 | | | Minimum inhibitory concentration (μg/ml) *1) | | |
|---|---|---|---|---|---|
| Ph | A | Salt | Candida albicans Ohta | Candida albicans MTU12001 | Trichophyton rubrum |
| 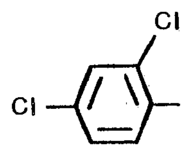 | 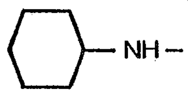 | Oxalate | 40 – 100 | 40 – 100 | 40 – 100 |
| 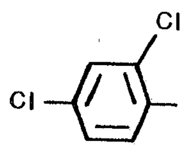 | 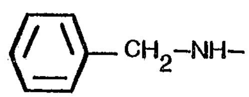 | Oxalate | 40 – 100 | 40 – 100 | 20 |
| 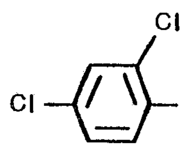 | 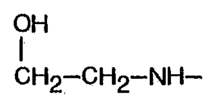 | Oxalate | 40 – 100 | 40 – 100 | 40 – 100 |
| 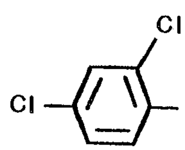 | 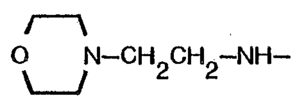 | Free | 40 – 100 | 40 – 100 | 40 – 100 |

TABLE 1 (Continued)

| Imidazolylpropanol Compound (I) 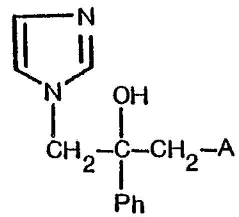 | | | Minimum inhibitory concentration (μg/ml) *1) | | |
|---|---|---|---|---|---|
| Ph | A | Salt | Candida albicans Ohta | Candida albicans MTU12001 | Trichophyton rubrum |
| 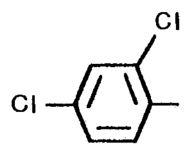 | 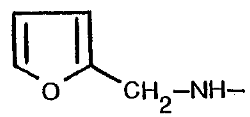 | Oxalate | 40 – 100 | 40 – 100 | 40 – 100 |
| 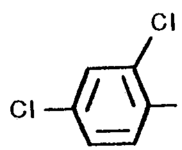 | 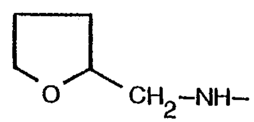 | Free | 40 – 100 | 40 – 100 | 40 – 100 |
| 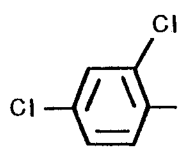 | 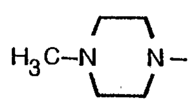 | Oxalate | 40 | 2.5 | 40 – 100 |
| 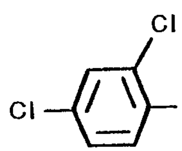 | 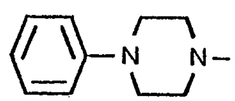 | Citrate | 20 | 5 | 0.6 |

TABLE 1 (Continued)

| Imidazolylpropanol Compound (I)<br>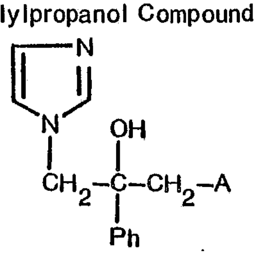 | | | Minimum inhibitory concentration (μg/ml) *1) | | |
| --- | --- | --- | --- | --- | --- |
| Ph | A | Salt | Candida albicans Ohta | Candida albicans MTU12001 | Trichophyton rubrum |
| 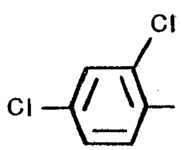 | 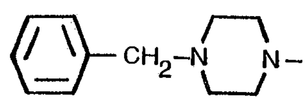 | Oxalate | 40 – 100 | 40 – 100 | 20 |

Note: *1) Sabouraud's agar medium was used.

The in vivo test of the antifungal activity with the said representative ones of the imidazolylpropanol compounds (I) was carried out in the following manner:

Candida albicans KB-8 cultured on the Sabroud's agar plate admixed with blood in a concentration of 5% by weight at 30°C for 4 days was suspended in physiologically saline solution to make a number of cells of $10^7$/ml. The suspension was intravenously injected through the tail vein into 5 week old male DDY mice at a dose of 0.2 ml per mouse.

The animals were orally medicated with the test compound in the form of 2% by weight methylcellulose suspension at a total dose of 20 mg per kg of the body weight immediately and 5 hours after the infection.

After 24 hours from the infection, the animals were killed and the kidneys were taken out. Each kidney was admixed with sea sand and smashed. After diluting with physiologically saline solution to give 5 times in volume, the dilution was centrifuged at 900 rpm for 5 minutes. A certain amount of the supernatant was scattered on a Sabroud's agar plate and cultured at 37°C for 48 hours. Then, the number of colonies was counted, the number of cells per one gram of the kidney was calculated, and the difference between the calculated numbers in the medicated group and in the non-medicated group was expressed by a logarithmic value. The results are shown below.

TABLE 2

Antifungal activity in vivo (Decrease of cells in the kidneys of Candida-infected mice)

| Imidazolylpropanol compound (I) 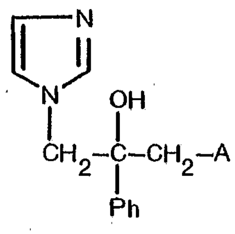 | | | Dose (per os, mg/kg) | Medicated group, number of cells in kidney (cell/g) | Non-medicated group, number of cells in kidney (cell/g) | Medicated/Non-medicated (logarithmic value) |
|---|---|---|---|---|---|---|
| Ph | A | Salt | | | | |
| 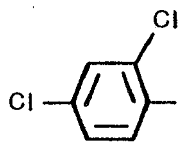 | $CH_3-(CH_2)_5-NH-$ | Free | 20 | $1.75 \times 10^3$ | $5.81 \times 10^4$ | –1.52 |
| 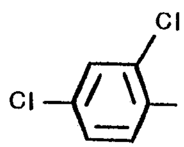 | 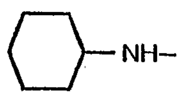 | Oxalate | 20 | $8.91 \times 10^3$ | $1.67 \times 10^5$ | –1.27 |
| 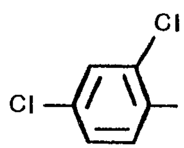 | 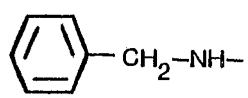 | Oxalate | 20 | $4.17 \times 10^3$ | $1.67 \times 10^5$ | –1.60 |

TABLE 2 (Continued)

| Imidazolylpropanol compound (I) 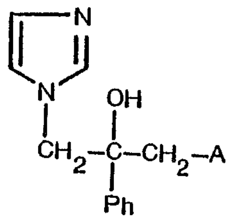 | | | Dose (per os, mg/kg) | Medicated group, number of cells in kidney (cell/g) | Non-medicated group, number of cells in kidney (cell/g) | Medicated/Non-medicated (logarithmic value) |
|---|---|---|---|---|---|---|
| Ph | A | Salt | | | | |
| 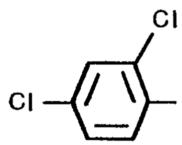 | 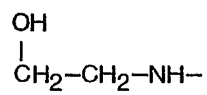 | Oxalate | 20 | $1.91 \times 10^3$ | $7.29 \times 10^4$ | –1.58 |
| 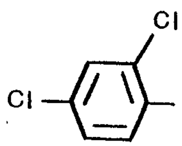 | 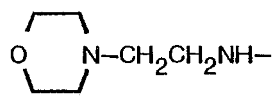 | Free | 20 | $9.78 \times 10^3$ | $1.67 \times 10^5$ | –1.23 |
| 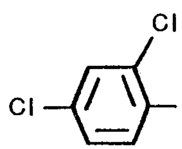 | 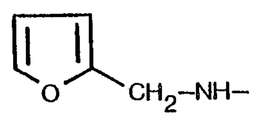 | Oxalate | 20 | $5.13 \times 10^3$ | $5.81 \times 10^4$ | –1.05 |
| 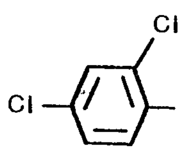 | 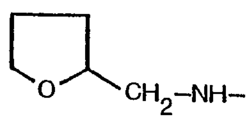 | Free | 20 | $5.62 \times 10^3$ | $5.81 \times 10^4$ | –1.01 |

TABLE 2 (Continued)

| Imidazolylpropanol compound (I)<br>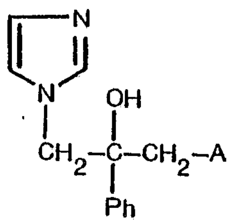 | | | Dose (per os, mg/kg) | Medicated group, number of cells in kidney (cell/g) | Non-medicated group, number of cells in kidney (cell/g) | Medicated/Non-medicated (logarithmic value) |
|---|---|---|---|---|---|---|
| Ph | A | Salt | | | | |
| 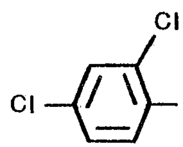 | 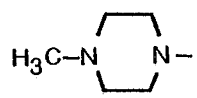 | Oxalate | 20 | $8.32 \times 10^3$ | $3.24 \times 10^5$ | –1.59 |
| 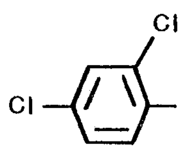 | 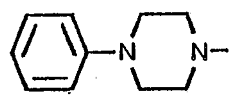 | Citrate | 20 | $1.52 \times 10^3$ | $5.81 \times 10^4$ | –1.58 |
| 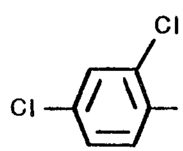 | 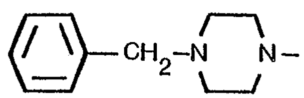 | Oxalate | 20 | $4.09 \times 10^3$ | $5.81 \times 10^4$ | –1.15 |
| Miconazole *1) | | | 100 | $2.96 \times 10^4$ | $1.86 \times 10^5$ | –0.80 |

Note: *1) 1-[2,4-Dichloro-β-(2,4-dichlorobenzyloxy)phenylethyl]imidazole (nitrate).

As can be seen from the above results, the imidazolylpropanol compounds (I) show an excellent antifungal activity.

Advantageously, the imidazolylpropanol compounds (I) are quite low in toxicity, and their $LD_{50}$ values were found to be more than 500 mg/kg when determined by oral route to mice. Thus, they are useful as antifungal agents.

The imidazolylpropanol compounds can be administered parenterally, orally or locally to warm-blooded animals and human beings in the form of conventional pharmaceutical preparations. For instance, they can be administered in the form of conventional solid pharmaceutical preparations such as tablets, capsules, powders or granules, or in the form of conventional liquid pharmaceutical preparations such as suspensions, emulsions or solutions. The daily dosage may vary depending upon the administration route and is usually between 10 mg to 5 g for human beings.

Practical embodiments of this invention are illustratively shown in the following examples.

### Example 1

To a solution of thiophenol (550 mg; 1.25 mol equivalent) in dimethylformamide (10 ml) kept at room temperature, 65% sodium hydride (185 mg; 1.25 mol equivalent) were added to make sodium thiophenoxide. 2-(2,4-Dichlorophenyl)-2-(imidazol-1-yl)methyloxirane (1.079 g; 1.0 mol equivalent) was added thereto, and the resultant mixture was stirred at room temperature for 3 hours. After completion of the reaction, the reaction mixture was distilled under reduced pressure to remove dimethylformamide and extracted with chloroform. The chloroform extract was washed with water, dried and concentrated. The residue was dissolved in ether, and fuming nitric acid was added thereto. The precipitated crystals were collected by filtration and recrystallized from methanol to give 2-(2,4-dichlorophenyl)-1-(imidazol-1-yl)-3-phenylthio-2-propanol nitrate (1.32 g) as colorless prisms melting at 158 to 160°C. Yield, 74.7%.

### Reference Example 1

To a suspension of 65% sodium hydride (5.55 g) in dimethylsulfoxide (200 ml), dimethyloxosulfonium methylide (33 g) was portionwise added, and the resultant mixture was stirred at room temperature for 30 minutes. A solution of 2,4-dichlorophenyl-(imidazol-1-yl)methylketone (25.5 g) in dimethylsulfoxide (50 ml) was dropwise added thereto, followed by stirring at 50°C for 3 hours. The reaction mixture was poured onto ice water (500 ml) and extracted with ether. The ether extract was washed with water, dried and concentrated. The residue was recrystallized from a mixture of ether and hexane to give 2-(2,4-dichlorophenyl)-2-(imidazol-1-yl)methyloxirane (14.7 g) as colorless prisms melting at 83 to 85°C. Yield, 54.6%.

### Example 2

To a solution of 3-methylthiophenol (322 mg) in dimethylformamide (5 ml) kept at room temperature, 65% sodium hydride (96 mg) were added to make sodium 3-methylthiophenoxide. 2-(2,4-Dichlorophenyl)-2-(imidazol-1-yl)methyloxirane (538 mg) was added thereto, and the resultant mixture was stirred at room temperature for 3 hours. After completion of the reaction, the reaction mixture was distilled under reduced pressure to remove dimethylformamide, and the residue was recrystallized from a mixture of acetone and ether to give 2-(2,4-dichlorophenyl)-1-(imidazol-1-yl)-3-(3-methylphenylthio)-2-propanol (570 mg) as colorless needles melting at 140 to 141.5°C. Yield, 72.5%.

### Examples 3 to 31

In the same manner as in Example 1 or 2, the following compounds were produced:

| Example No. | Product | Physical data |
|---|---|---|
| 3 | 2-(2,4-Dichlorophenyl)-1-(imidazol-1-yl)-3-(4-methoxyphenylthio)-2-propanol | M.P., 137—138.5°C |
| 4 | 2-(2,4-Dichlorophenyl)-3-(4-fluorophenylthio)-1-(imidazol-1-yl)-2-propanol | M.P., 161.5—163°C |
| 5 | 3-(4-Chlorophenylthio)-2-(2,4-dichlorophenyl)-1-(imidazol-1-yl)-2-propanol | M.P., 147—148°C |
| 6 | 3-(4-Bromophenylthio)-2-(2,4-dichlorophenyl)-1-(imidazol-1-yl)-2-propanol | M.P., 140—141°C |
| 7 | 2-(2,4-Dichlorophenyl)-3-(2,4-dichlorophenylthio)-1-(imidazol-1-yl)-2-propanol | M.P., 138—139°C |

| Example No. | Product | Physical data |
|---|---|---|
| 8 | 2-(2,4-Dichlorophenyl)-3-(2,5-dichlorophenylthio)-1-(imidazol-1-yl)-2-propanol | M.P., 161—162°C |
| 9 | 3-(Benzylthio)-2-(2,4-dichlorophenyl)-1-(imidazol-1-yl)-2-propanol | M.P., 128—130°C |
| 10 | 3-(n-Butylthio)-2-(2,4-dichlorophenyl)-1-(imidazol-1-yl)-2-propanol | M.P., 90—92°C |
| 11 | 2-(2,4-Dichlorophenyl)-1-(imidazol-1-yl)-3-(4-pyridylthio)-2-propanol (nitrate) | M.P., 167—169°C |
| 12 | 2-(2,4-Dichlorophenyl)-1-(imidazol-1-yl)-3-(n-propylthio)-2-propanol | M.P. 130—138°C |
| 13 | 2-(2,4-Dichlorophenyl)-1-(imidazol-1-yl)-3-(isopropylthio)-2-propanol | M.P., 140—141°C |
| 14 | 3-(n-Butylthio)-2-(2,4-dichlorophenyl)-1-(imidazol-1-yl)-2-propanol (hydrochloride) | M.P., 143—144°C |
| 15 | 3-(sec-Butylthio)-2-(2,4-dichlorophenyl)-1-(imidazol-1-yl)-2-propanol | M.P., 113—114°C |
| 16 | 3-(Isobutylthio)-2-(2,4-dichlorophenyl)-1-(imidazol-1-yl)-2-propanol | M.P., 130—132°C |
| 17 | 3-(tert-Butylthio)-2-(2,4-dichlorophenyl)-1-(imidazol-1-yl)-2-propanol | M.P., 196—197°C |
| 18 | 2-(2,4-Dichlorophenyl)-1-(imidazol-1-yl)-(n-pentylthio)-2-propanol | M.P., 104—105°C |
| 19 | 3-(tert-Amylthio)-2-(2,4-dichlorophenyl)-1-(imidazol-1-yl)-2-propanol | M.P., 174—175°C |
| 20 | 3-(Isoamylthio)-2-(2,4-dichlorophenyl)-1-(imidazol-1-yl)-2-propanol | M.P., 152—154°C |
| 21 | 2-(2,4-Dichlorophenyl)-3-(n-hexylthio)-1-(imidazol-1-yl)-2-propanol | M.P., 104—105°C |
| 22 | 2-(2,4-Dichlorophenyl)-3-(n-heptylthio)-1-(imidazol-1-yl)-2-propanol | M.P., 81—82°C |
| 23 | 2-(2,4-Dichlorophenyl)-1-(imidazol-1-yl)-3-(n-octylthio)-2-propanol | M.P., 76—78°C |
| 24 | 2-(2,4-Dichlorophenyl)-1-(imidazol-1-yl)-3-(n-nonylthio)-2-propanol | M.P., 70—72°C |
| 25 | 3-(n-Decanoylthio)-2-(2,4-dichlorophenyl)-1-(imidazol-1-yl)-2-propanol | M.P., 70—72°C |
| 26 | 2-(2,4-Dichlorophenyl)-3-(n-dodecylthio)-1-(imidazol-1-yl)-2-propanol (oxalate) | M.P., 128—129°C |
| 27 | 2-(2,4-Dichlorophenyl)-1-(imidazol-1-yl)-3-(n-octadecylthio)-2-propanol | M.P., 68—72°C |
| 28 | 2-(2,4-Dichlorophenyl)-1-(imidazol-1-yl)-3-(tert-nonylthio)-2-propanol | M.P., 90—92°C |

| Example No. | Product | Physical data |
|---|---|---|
| 29 | 3-(n-Butylthio)-2-(2-chlorophenyl)-1-(imidazol-1-yl)-2-propanol | M.P., 74—75°C |
| 30 | 2-(2-Chlorophenyl)-1-(imidazol-1-yl)-3-(n-pentylthio)-2-propanol (oxalate) | M.P., 139—142°C |
| 31 | 2-(2-Chlorophenyl)-3-(n-hexylthio)-1-(imidazol-1-yl)-2-propanol (oxalate) | M.P., 143—147°C |

Example 32

65% Sodium hydride (74 mg) were portionwise added to n-butanol. After the generation of hydrogen gas ceased, 2-(2,4-dichlorophenyl)-2-(imidazol-1-yl)methyloxirane (269 mg) was added thereto, and the resultant mixture was refluxed for 3.5 hours. After removal of n-butanol by distillation, the residue was extracted with chloroform, and the chloroform extract was washed with water, dried and concentrated. The oily residue was recrystallized from n-hexane to give 3-n-butyloxy-2-(2,4-dichlorophenyl)-1-(imidazol-1-yl)-2-propanol (120 mg) as crystals melting at 94 to 98°C. Yield, 34.9%.

Examples 33 to 41

In the same manner as in Example 32, the following compounds were produced:

| Example No. | Product | Physical data |
|---|---|---|
| 33 | 2-(2,4-Dichlorophenyl)-3-ethoxy-1-(imidazol-1-yl)-2-propanol | M.P., 108—112°C |
| 34 | 2-(2,4-Dichlorophenyl)-1-(imidazol-1-yl)-3-isoamyloxy-2-propanol (oxalate) | M.P., 60—65°C |
| 35 | 3-Allyloxy-2-(2,4-dichlorophenyl)-1-(imidazol-1-yl)-2-propanol (oxalate) | M.P., 134—145°C |
| 36 | 2-(2,4-Dichlorophenyl)-1-(imidazol-1-yl)-3-undecyloxy-2-propanol (oxalate) | M.P., 91—98°C |
| 37 | 2-(2,4-Dichlorophenyl)-1-(imidazol-1-yl)-3-(2-n-propylthioethyloxy)-2-propanol (oxalate) | M.P., 113—116°C |
| 38 | 3-Benzyloxy-2-(2,4-dichlorophenyl)-1-imidazol-1-yl)-2-propanol (oxalate) | M.P., 85—90°C |
| 39 | 2-(2,4-Dichlorophenyl)-1-(imidazol-1-yl)-3-(o-fluorobenzyloxy)-2-propanol (oxalate) | M.P., 80—83°C |
| 40 | 2-(2,4-Dichlorophenyl)-1-(imidazol-1-yl)-3-(2-phenylethyloxy)-2-propanol (oxalate) | M.P., 82—93°C |
| 41 | 2-(2,4-Dichlorophenyl)-1-(imidazol-1-yl)-3-(2-phenylthioethyloxy)-2-propanol (oxalate) | M.P., 48—56°C |

Example 42

To a solution prepared from lithium hydroxide hydrate (94 mg), water (3 ml), phenol (227 mg) and benzyltrimethylammonium chloride (35 mg), 2-(2,4-dichlorophenyl)-2-(imidazol-1-yl)methyloxirane (500 mg) was added, and the resultant mixture was stirred at 95 to 100°C for 5 hours. The reaction mixture was diluted with water and extracted with ether. The ether extract was washed with water, dried and concentrated. The residue was purified by silica gel chromatography and treated with nitric acid. Recrystallization from ethanol gave 2-(2,4-dichlorophenyl)-1-(imidazol-1-yl)-3-phenoxy-2-propanol nitrate (250 mg) as crystals melting at 149 to 151°C. Yield, 32%. The compound prepared in this example is not claimed herein.

Example 43

In the same manner as in Example 42, the following compound was produced:

| Product | Physical data |
|---|---|
| 2-(2,4-Dichlorophenyl)-1-(imidazol-1-yl)-2,3-propanediol | M.P., 155—158°C |

Reference Example 2

To a suspension of 50% sodium hydride (147 mg) in dimethylsulfoxide (4 ml) kept at room temperature, dimethyloxosulfonium methylide (674 mg) was portionwise added, and the resultant mixture was stirred for 15 minutes. After addition of phenoxymethyl phenyl ketone (500 mg), stirring was further continued for 12 hours. The reaction mixture was admixed with water and extracted with ether. The ether extract was concentrated and purified by column chromatography to give 2-phenoxymethyl-2-phenyloxirane (350 mg) as an oil.

Yield, 66 %. Mass spectrum: M/e, 226 ($M^+$).

NMR spectrum (in $CDCl_3$): $\delta$, 2.70, 2.93 (each 1H, d, $J_{gem}$ = 8.5H 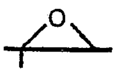 $CH_2$), 4.24 (2H, s, –O–$CH_2$– 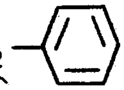), 6.6 – 7.5 (10H, aromatic proton).

Example 44

In a sealed tube, a solution of 2-(2,4-dichlorophenyl)-2-(imidazol-1-yl)methyloxirane (1.0 g) in n-propylamine (10 ml) was heated at 100°C for 15 hours. After excessive n-propylamine was eliminated by distillation, the residue was extracted with chloroform. The chloroform extract was washed with water, dried and concentrated. The oily residue (1.35 g) was purified by silica gel chromatography. The resulting product was recrystallized from a mixture of n-hexane and ether to give 2-(2,4-dichlorophenyl)-1-(imidazol-1-yl)-3-n-propylamino-2-propanol (584 mg) as colorless prisms melting at 85 to 86°C. Yield, 48%.

Example 45

To a soolution of 2-(2,4-dichlorophenyl)-2-(imidazol-1-yl)methyloxirane (1.0 g) in methanol (10 ml), morpholine (2 ml) was added, and the resultant mixture was refluxed for 5 hours. After removal of the solvent by distillation, the residue was extracted with ether. The ether extract was washed with water, dried and concentrated. The oily residue was purified by silica gel chromatography to give 2-(2,4-dichlorophenyl)-1-(imidazol-1-yl)-3-morpholino-2-propanol (850 mg) as an oil. Yield, 64%.

The oil was dissolved in ether, and oxalic acid was added thereto to make pH 3—4. The precipitated crystals were collected by filtration to give the oxalate as colorless needles melting at 147 to 150°C.

Examples 46 to 58

In the same manner as in Example 45 or 46, the following compounds were produced:

| Example No. | Product | Physical data |
|---|---|---|
| 46 | 2-(2,4-Dichlorophenyl)-3-(di-n-propylamino)-1-(imidazol-1-yl)-2-propanol (oil) | $n_D^{22}$ 1.5462 |
| 47 | 2-(2,4-Dichlorophenyl)-3-(n)-hexylamino-1-(imidazol-1-yl)-2-propanol | M.P., 153—154.5°C |
| 48 | 3-Cyclohexylamino-2-(2,4-di-chlorophenyl)-1-(imidazol-1-yl)-2-propanol (oxalate) | M.P., 110—125°C |
| 49 | 3-Benzylamino-2-(2,4-dichlorophenyl)-1-(imidazol-1-yl)-2-propanol (oxalate) | M.P., 104—106°C |
| 50 | 2-(2,4-Dichlorophenyl)-3-(2-hydroxyethylamino)-1-(imidazol-1-yl)-2-propanol (oxalate) | M.P., 177—178°C |
| 51 | 2-(2,4-Dichlorophenyl)-1-(imidazol-1-yl)-3-(2-morpholinoethylamino)-2-propanol | M.P., 131—132°C |

| Example No. | Product | Physical data |
|---|---|---|
| 52 | 2-(2,4-Dichlorophenyl)-1-(imidazol-1-yl)-3-(2-methoxyethylamino)-2-propanol (oil) | $n_D^{23}$ 1.5587 |
| 53 | 2-(2,4-Dichlorophenyl)-1-(imidazol-1-yl)-3-propargylamino-2-propanol | M.P., 95—97°C |
| 54 | 2-(2,4-Dichlorophenyl)-3-furfurylamino-1-(imidazol-1-yl)-2-propanol (oxalate) | M.P., 87—90°C |
| 55 | 2-(2,4-Dichlorophenyl)-1-(imidazol-1-yl)-3-tetrahydrofurfurylamino-2-propanol | M.P., 148.5—155.5°C |
| 56 | 2-(2,4-Dichlorophenyl)-1-(imidazol-1-yl)-3-(N-methylpiperazino)-2-propanol (oxalate) | M.P., 173—176°C |
| 57 | 2-(2,4-Dichlorophenyl)-1-(imidazol-1-yl)-3-(N-phenylpiperazino))-2-propanol (citrate) | M.P., 89—91°C |
| 58 | 2-(2,4-Dichlorophenyl)-1-(imidazol-1-yl)-3-(N-benzylpiperazino)-2-propanol (oxalate) | M.P., 104—106°C |

In the similar manner as above, the following compounds are obtained:
3-(p-Chlorobenzylamino)-2-(2,4-dichlorophenyl)-1-(imidazol-1-yl)-2-propanol;
2-(2,4-Dichlorophenyl)-1-(imidazol-1-yl)-3-piperazino-2-propanol;
2-(2,4-Dichlorophenyl)-1-(imidazol-1-yl)-3-pyrrolidino-2-propanol;
2-(2,4-Dichlorophenyl)-1-(imidazol-1-yl)-3-piperazino-2-propanol.

**Claims for the Contracting States: BE CH DE FR GB IT LI NL SE**

1. An imidazolylpropanol compound of the formula:

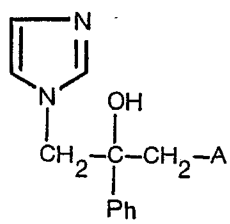

(I)

wherein

Ph is a phenyl group or a phenyl group substituted with halogen; and

A is —S—$R^1$ (wherein $R^1$ is a $C_1$—$C_{15}$ or a $C_{18}$ alkyl group, a phenyl group, a phenyl group substituted with at least one of $C_1$—$C_8$ alkyl, $C_1$—$C_8$ alkoxy and halogen, a benzyl group or a pyridyl group),

—O—$R^2$ (wherein $R^2$ is a hydrogen atom, a $C_1$—$C_{15}$ alkyl group, a $C_1$—$C_8$ alkyl group substituted with at least one of phenylthio and $C_1$—$C_8$ alkylthio, a $C_3$—$C_8$ alkenyl group, a $C_7$—$C_8$ aralkyl group, a $C_7$—$C_8$ aralkyl group substituted with halogen), or

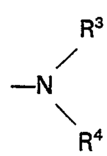

(wherein $R^3$ and $R^4$ are each a hydrogen atom, a $C_1$—$C_8$ alkyl group, a $C_3$—$C_6$ cycloalkyl group, a benzyl group, a benzyl group substituted with halogen, a $C_1$—$C_8$ alkyl group substituted with at least one of hydroxyl, morpholino and $C_1$—$C_8$ alkoxy, a $C_3$—$C_6$ alkynyl group, a furfuryl group or a tetrahydrofurfuryl group, or when taken together with the adjacent nitrogen atom, they represent a morpholino group, a piperazino group, an N-($C_1$—$C_8$)alkylpiperazino group, an N-phenylpiperazino group, an N-benzylpiperazino group, a pyrrolidino group or a piperidino group),
and its acid addition salts.

2. The compound according to claim 1, wherein A is —S—$R^1$.
3. The compound according to claim 1, wherein A is —O—$R^2$.
4. The compound according to claim 1, wherein A is

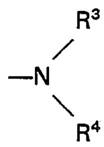

5. The compound according to claim 2, wherein Ph is a phenyl group substituted with halogen and A is a $C_1$—$C_{15}$ alkylthio group.

6. The compound according to claim 2, wherein Ph is a 2,4-dichlorophenyl group and A is a phenylthio group.

7. The compound according to claim 2, wherein Ph is a 2,4-dichlorophenyl group and A is a 3-methylphenylthio group.

8. The compound according to claim 2, wherein Ph is a 2,4-dichlorophenyl group and A is a 4-methoxyphenylthio group.

9. The compound according to claim 2, wherein Ph is a 2,4-dichlorophenyl group and A is a 4-fluorophenylthio group.

10. The compound according to claim 2, wherein Ph is a 2,4-dichlorophenyl group and A is a benzylthio group.

11. The compound according to claim 5, wherein Ph is a 2,4-dichlorophenyl group and A is an n-butylthio group.

12. The compound according to claim 2, wherein Ph is a 2,4-dichlorophenyl group and A is a 4-pyridylthio group.

13. The compound according to claim 3, wherein Ph is a 2,4-dichlorophenyl group and A is an n-butoxy group.

14. The compound according to claim 3, wherein Ph is a 2,4-dichlorophenyl group and A is an isoamyloxy group.

15. The compound according to claim 4, wherein Ph is a 2,4-dichlorophenyl group and A is an n-hexylamino group.

16. The compound according to claim 4, wherein Ph is a 2,4-dichlorophenyl group and A is a benzylamino group.

17. The compound according to claim 4, wherein Ph is a 2,4-dichlorophenyl group and A is a 2-hydroxyethylamino group.

18. The compound according to claim 4, wherein Ph is a 2,4-dichlorophenyl group and A is an N-methylpiperazino group.

19. The compound according to claim 4, wherein Ph is a 2,4-dichlorophenyl group and A is an N-phenylpiperazino group.

20. A process for preparing imidazolylpropanol compounds of the formula (I) as set forth in claim 1 and their acid addition salts, which comprises reacting either

a) an epoxide compound of the formula

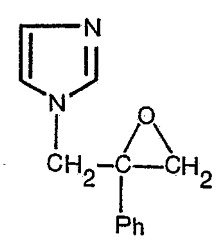

(II)

wherein Ph is as defined in claim 1 with a reagent of either one of the formulas:

$\overset{\oplus\ominus}{MS}-R^1$ (IIIa)

$\overset{\oplus\ominus}{MO}-R^2$ (IIIb)

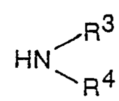

(IIIc)

wherein M is an alkali metal atom and $R^1$, $R^2$, $R^3$ and $R^4$ are each as defined in claim 1, or

b) an epoxide compound of the formula

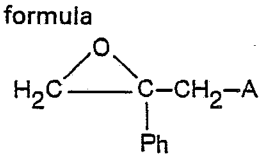

(IV)

wherein Ph and A are each as defined in claim 1 with an imidazole compound of the formula:

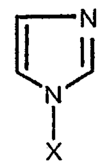

(V)

wherein X is potassium or sodium.

21. An antifungal composition which comprises an antifungally effective amount of at least one of the imidazolylpropanol compounds and their acid addition salts according to claim 1, as an active ingredient and at least one inert carrier or diluent.

22. Imidazolylpropanol compounds and their acid addition salts according to claim 1 for use as antifungal agents.

**Claims for the Contracting State: AT**

1. A process for preparing imidazolylpropanol compounds of the formula:

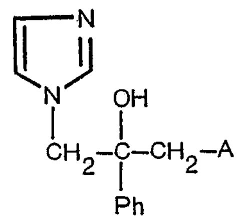

(I)

wherein

Ph is a phenyl group or a phenyl group substituted with halogen; and

A is —S—$R^1$ (wherein $R^1$ is a $C_1$—$C_{15}$ or a $C_{18}$ alkyl group, a phenyl group, a phenyl group substituted with at least one of $C_1$—$C_8$ alkyl, $C_1$—$C_8$ alkoxy and halogen, a benzyl group or a pyridyl group),

—O—$R^2$ (wherein $R^2$ is a hydrogen atom, a $C_1$—$C_{15}$ alkyl group, a $C_1$—$C_8$ alkyl group substituted with at least one of phenylthio and $C_1$—$C_8$ alkylthio, a $C_3$—$C_8$ alkenyl group, a $C_7$—$C_8$ aralkyl group, a $C_7$—$C_8$ aralkyl group substituted with halogen, or

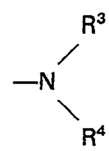

(wherein $R^3$ and $R^4$ are each a hydrogen atom, a $C_1$—$C_8$ alkyl group, a $C_3$—$C_6$ cycloalkyl group, a benzyl group, a benzyl group substituted with halogen, a $C_1$—$C_8$ alkyl group substituted with at least one of hydroxyl, morpholino and $C_1$—$C_8$ alkoxy, a $C_3$—$C_6$ alkynyl group, a furfuryl group or a tetrahydrofurfuryl group, or when taken together with the adjacent nitrogen atom, they represent a morpholino group, a piperazino group, an N-($C_1$—$C_8$)alkylpiperazino group, an N-phenylpiperazino group, an N-benzylpiperazino group, a pyrrolidino group or a piperidino group),

and its acid addition salts, which comprises reacting either

a) an epoxide compound of the formula

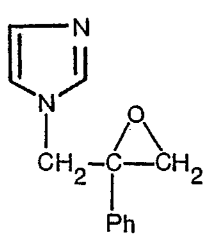

(II)

wherein Ph is as defined in claim 1 with a reagent of either one of the formulas:

$\overset{\oplus\ominus}{MS}-R^1$ (IIIa)

$\overset{\oplus\ominus}{MO}-R^2$ (IIIb)

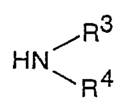

(IIIc)

wherein M is an alkali metal atom and $R^1$, $R^2$, $R^3$ and $R^4$ are each as defined above or

b) an epoxide compound of the formula

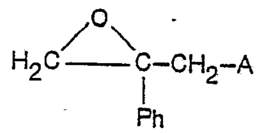

(IV)

wherein Ph and A are each as defined in claim 1 with an imidazole compound of the formula:

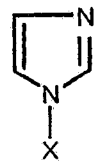

(V)

wherein X is potassium or sodium.

2. The process for preparing a compound according to claim 1, wherein A is $—S—R^1$.

3. The process for preparing a compound according to claim 1, wherein A is $—O—R^2$.

4. The process for preparing a compound according to claim 1, wherein A is

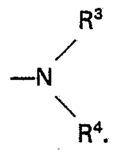

5. The process for preparing a compound according to claim 2, wherein Ph is a phenyl group substituted with halogen and A is a $C_1$—$C_{15}$ alkylthio group.

6. The process for preparing a compound according to claim 2, wherein Ph is a 2,4-dichlorophenyl group and A is a phenylthio group.

7. The process for preparing a compound according to claim 2, wherein Ph is a 2,4-dichlorophenyl group and A is a 3-methylphenylthio group.

8. The process for preparing a compound according to claim 2, wherein Ph is a 2,4-dichlorophenyl group and A is a 4-methoxyphenylthio group.

9. The process for preparing a compound according to claim 2, wherein Ph is a 2,4-dichlorophenyl group and A is a 4-fluorophenylthio group.

10. The process for preparing a compound according to claim 2, wherein Ph is a 2,4-dichlorophenyl group and A is a benzylthio group.

11. The process for preparing a compound according to claim 5, wherein Ph is a 2,4-dichlorophenyl group and A is an n-butylthio group.

12. The process for preparing a compound according to claim 2, wherein Ph is a 2,4-dichlorophenyl group and A is a 4-pyridylthio group.

13. The process for preparing a compound according to claim 3, wherein Ph is a 2,4-dichlorophenyl group and A is an n-butoxy group.

14. The process for preparing a compound according to claim 3, wherein Ph is a 2,4-dichlorophenyl group and A is an isoamyloxy group.

15. The process for preparing a compound according to claim 4, wherein Ph is a 2,4-dichlorophenyl group and A is an n-hexylamino group.

16. The process for preparing a compound according to claim 4, wherein Ph is a 2,4-dichlorophenyl group and A is a benzylamino group.

17. The process for preparing a compound according to claim 4, wherein Ph is a 2,4-dichlorophenyl group and A is a 2-hydroxyethylamino group.

18. The process for preparing a compound according to claim 4, wherein Ph is a 2,4-dichlorophenyl group and A is an N-methylpiperazino group.

19. The process for preparing a compound according to claim 4, wherein Ph is a 2,4-dichlorophenyl group and A is an N-phenylpiperazino group.

20. A process for preparing an antifungal composition which comprises the formulation of an antifungally effective amount of at least one of the imidazolylpropanol compounds and their acid addition salts according to claim 1 as an active ingredient with at least one inert carrier or diluent.

**Patentansprüche für die Vertragsstaaten: BE CH DE FR GB IT LI NL SE**

1. Imidazolylpropanolverbindung der Formel:

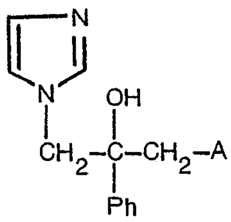

(I)

worin

Ph eine Phenylgruppe oder eine durch Halogen substituierte Phenylgruppe ist; und

A für —S—$R^1$ (worin $R^1$ eine Alkylgruppe mit 1 bis 15 Kohlenstoffatomen oder 18 Kohlenstoffatomen, eine Phenylgruppe, eine durch mindestens einen der Substituenten Alkyl mit 1 bis 8 Kohlenstoffatomen, Alkoxy mit 1 bis 8 Kohlenstoffatomen und Halogen substituierte Phenylgruppe, eine Benzylgruppe oder eine Pyridylgruppe ist),

—O—$R^2$ (worin $R^2$ ein Wasserstoffatom, eine Alkylgruppe mit 1 bis 15 Kohlenstoffatomen, eine durch mindestens einen der Substituenten Phenylthio und Alkylthio mit 1 bis 8 Kohlenstoffatomen substituierte Alkylgruppe mit 1 bis 8 Kohlenstoffatomen, eine Alkenylgruppe mit 3 bis 8 Kohlenstoffatomen, eine Aralkylgruppe mit 7 oder 8 Kohlenstoffatomen oder eine durch Halogen substituierte Aralkylgruppe mit 7 oder 8 Kohlenstoffatomen ist), oder

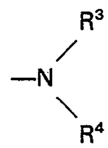

(worin $R^3$ und $R^4$ jeweils ein Wasserstoffatomen, eine Alkylgruppe mit 1 bis 8 Kohlenstoffatomen, eine Cycloalkylgruppe mit 3 bis 6 Kohlenstoffatomen, eine Benzylgruppe, eine durch Halogen substituierte Benzylgruppe, eine durch mindestens einen der Substituenten Hydroxyl, Morpholino und Alkoxy mit 1 bis 8 Kohlenstoffatomen substituierte Alkylgruppe mit 1 bis 8 Kohlenstoffatomen, eine Alkinylgruppe mit 3 bis 6 Kohlenstoffatomen, eine Furfurylgruppe oder eine Tetrahydrofurfurylgruppe sind oder zusammen mit dem benachbarten Stickstoffatom eine Morpholinogruppe, eine Piperazinogruppe, eine N-Alkylpiperazinogruppe mit 1 bis 8 Kohlenstoffatomen im Alkylrest, eine N-Phenylpiperazinogruppe, eine N-Benzylpiperazinogruppe, eine Pyrrolidinogruppe oder eine Piperidinogruppe darstellen)
steht, und deren Säureadditionssalze.

2. Verbindung nach Anspruch 1, worin A für —S—$R^1$ steht.

3. Verbindung nach Anspruch 1, worin A für —O—$R^2$ steht.

4. Verbindung nach Anspruch 1, worin A für

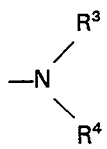

steht.

5. Verbindung nach Anspruch 2, worin Ph eine durch Halogen substituierte Phenylgruppe ist und A eine Alkylthiogruppe mit 1 bis 15 Kohlenstoffatomen ist.

6. Verbindung nach Anspruch 2, worin Ph eine 2,4-Dichlorphenylgruppe ist und A eine Phenylthiogruppe ist.

7. Verbindung nach Anspruch 2, worin Ph eine 2,4-Dichlorphenylgruppe ist und A eine 3-Methylphenylthiogruppe ist.

8. Verbindung nach Anspruch 2, worin Ph eine 2,4-Dichlorphenylgruppe ist und A eine 4-Methoxyphenylthiogruppe ist.

9. Verbindung nach Anspruch 2, worin Ph eine 2,4-Dichlorphenylgruppe ist und A eine 4-Fluorphenylthiogruppe ist.

10. Verbindung nach Anspruch 2, worin Ph eine 2,4-Dichlorphenylgruppe ist und A eine Benzylthiogruppe ist.

11. Verbindung nach Anspruch 5, worin Ph eine 2,4-Dichlorphenylgruppe ist und A eine n-Butylthiogruppe ist.

12. Verbindung nach Anspruch 2, worin Ph eine 2,4-Dichlorphenylgruppe ist und A eine 4-Pyridylthiogruppe ist.

13. Verbindung nach Anspruch 3, worin Ph eine 2,4-Dichlorphenylgruppe ist und A eine n-Butoxygruppe ist.

14. Verbindung nach Anspruch 3, worin Ph eine 2,4-Dichlorphenylgruppe ist und A eine Isoamyloxygruppe ist.

15. Verbindung nach Anspruch 4, worin Ph eine 2,4-Dichlorphenylgruppe ist und A eine n-Hexylaminogruppe ist.

16. Verbindung nach Anspruch 4, worin Ph eine 2,4-Dichlorphenylgruppe ist und A eine Benzylaminogruppe ist.

17. Verbindung nach Anspruch 4, worin Ph eine 2,4-Dichlorphenylgruppe ist und A eine 2-Hydroxyethylaminogruppe ist.

18. Verbindung nach Anspruch 4, worin Ph eine 2,4-Dichlorphenylgruppe ist und A eine N-Methylpiperazinogruppe ist.

19. Verbindung nach Anspruch 4, worin Ph eine 2,4-Dichlorphenylgruppe ist und A eine N-Phenylpiperazinogruppe ist.

20. Verfahren zur Herstellung von Imidazolylpropanolverbindungen der in Anspruch 1 angegebenen Formel I und deren Säureadditionssalzen, dadurch gekennzeichnet, dass man entweder

a) eine Epoxidverbindung der Formel:

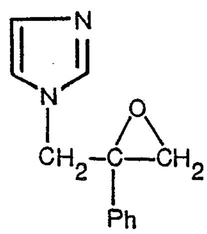

(II)

worin Ph wie im Anspruch 1 definiert ist, mit einem Reagenz einer der Formeln:

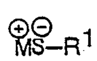

(IIIa)

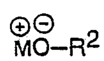

(IIIb)

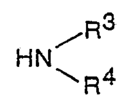

(IIIc)

umsetzt, worin M ein Alkalimetallatom ist und $R^1$, $R^2$, $R^3$ und $R^4$ jeweils wie in Anspruch 1 definiert sind, oder

b) eine Epoxydverbindung der Formel:

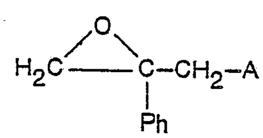

(IV)

worin Ph und A jeweils wie in Anspruch 1 definiert sind, mit einer Imidazolverbindung der Formel:

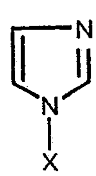

(V)

umsetzt, worin X Kalium oder Natrium ist.

21. Antifungale Zubereitung, dadurch gekennzeichnet, dass sie eine antifungal wirksame Menge mindestens einer der Imidazolylpropanolverbindungen und ihrer Säureadditionssalze gemäss Anspruch 1 als Wirkstoffkomponente und mindestens einen inerten Träger und/oder mindestens ein inertes Verdünnungsmittel enthält.

22. Imidazolylpropanolverbindungen und deren Säureadditionssalze gemäss Anspruch 1 für die Verwendung als antifungale Mittel.

**Patentansprüche für den Vertragsstaat: AT**

1. Verfahren zur Herstellung von Imidazolylpropanolverbindungen der Formel:

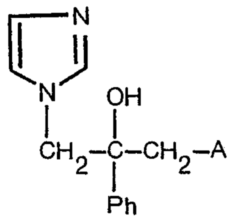

(I)

worin

Ph eine Phenylgruppe oder eine durch Halogen substituierte Phenylgruppe ist; und

A für —S—$R^1$ (worin $R^1$ eine Alkylgruppe mit 1 bis 15 Kohlenstoffatomen oder 18 Kohlenstoffatomen, eine Phenylgruppe, eine durch mindestens einen der Substituenten Alkyl mit 1 bis 8 Kohlenstoffatomen, Alkoxy mit 1 bis 8 Kohlenstoffatomen und Halogen substituierte Phenylgruppe, eine Benzylgruppe oder eine Pyridylgruppe ist),

—O—$R^2$ (worin $R^2$ ein Wasserstoffatom, eine Alkylgruppe mit 1 bis 15 Kohlenstoffatomen, eine durch mindestens einen der Substituenten Phenylthio und Alkylthio mit 1 bis 8 Kohlenstoffatomen substituierte Alkylgruppe mit 1 bis 8 Kohlenstoffatomen, eine Alkenylgruppe mit 3 bis 8 Kohlenstoffatomen, eine Aralkylgruppe mit 7 oder 8 Kohlenstoffatomen oder eine durch Halogen substituierte Aralkylgruppe mit 7 oder 8 Kohlenstoffatomen ist), oder

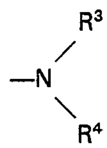

(worin $R^3$ und $R^4$ jeweils ein Wasserstoffatomen, eine Alkylgruppe mit 1 bis 8 Kohlenstoffatomen, eine Cycloalkylgruppe mit 3 bis 6 Kohlenstoffatomen, eine Benzylgruppe, eine durch Halogen substituierte Benzylgruppe, eine durch mindestens einen der Substituenten Hydroxyl, Morpholino und Alkoxy mit 1 bis 8 Kohlenstoffatomen substituierte Alkylgruppe mit 1 bis 8 Kohlenstoffatomen, eine Alkinylgruppe mit 3 bis 6 Kohlenstoffatomen, eine Furfurylgruppe oder eine Tetrahydrofurfurylgruppe sind oder zusammen mit dem benachbarten Stickstoffatom eine Morpholinogruppe, eine Piperazinogruppe, eine N-Alkylpiperazinogruppe mit 1 bis 8 Kohlenstoffatomen im Alkylrest, eine N-Phenylpiperazinogruppe, eine N-Benzylpiperazinogruppe, eine Pyrrolidinogruppe oder eine Piperidinogruppe darstellen)

steht, und deren Säureadditionssalzen, dadurch gekennzeichnet, dass man entweder

a) eine Expoxidverbindung der Formel:

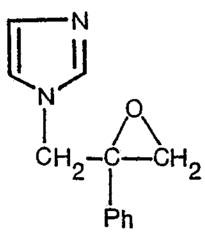

(II)

worin Ph wie oben efiniert ist, mit einem Reagenz einer der Formeln:

$\overset{\oplus\ominus}{MS}-R^1$ (IIIa)

$\overset{\oplus\ominus}{MO}-R^2$ (IIIb)

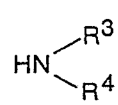

(IIIc)

umsetzt, worin M ein Alkalimetallatom ist und $R^1$, $R^2$, $R^3$ und $R^4$ jeweils wie oben definiert sind, oder

b) eine Epoxydverbindung der Formel:

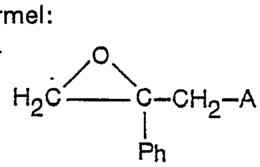

(IV)

worin Ph und A jeweils wie oben definiert sind, mit einer Imidazolverbindung der Formel:

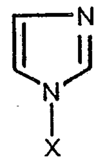

(V)

umsetzt, worin X Kalium oder Natrium ist.

2. Verfahren zur Herstellung einer Verbindung nach Anspruch 1, worin A für —S—$R^1$ steht.

3. Verfahren zur Herstellung einer Verbindung nach Anspruch 1, worin A für —O—$R^2$ steht.

4. Verfahren zur Herstellung einer Verbindung nach Anspruch 1, worin A für

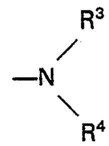

steht.

5. Verfahren zur Herstellung einer Verbindung nach Anspruch 2, worin Ph eine durch Halogen substituierte Phenylgruppe ist und A eine Alkylthiogruppe mit 1 bis 15 Kohlenstoffatomen ist.

6. Verfahren zur Herstellung einer Verbindung nach Anspruch 2, worin Ph eine 2,4-Dichlorphenylgruppe ist und A eine Phenylthiogruppe ist.

7. Verfahren zur Herstellung einer Verbindung nach Anspruch 2, worin Ph eine 2,4-Dichlorphenylgruppe ist und A eine 3-Methylphenylthiogruppe ist.

8. Verfahren zur Herstellung einer Verbindung nach Anspruch 2, worin Ph eine 2,4-Dichlorphenylgruppe ist und A eine 4-Methoxypenylthiogruppe ist.

9. Verfahren zur Herstellung einer Verbindung nach Anspruch 2, worin Ph eine 2,4-Dichlorphenylgruppe ist und A eine 4-Fluorphenylthiogruppe ist.

10. Verfahren zur Herstellung einer Verbindung nach Anspruch 2, worin Ph eine 2,4-Dichlorphenylgruppe ist und A eine Benzylthiogruppe ist.

11. Verfahren zur Herstellung einer Verbindung nach Anspruch 5, worin Ph eine 2,4-Dichlorphenylgruppe ist und A eine n-Butylthiogruppe ist.

12. Verfahren zur Herstellung einer Verbindung nach Anspruch 2, worin Ph eine 2,4-Dichlorphenylgruppe ist und A eine 4-Pyridylthiogruppe ist.

13. Verfahren zur Herstellung einer Verbindung nach Anspruch 3, worin Ph eine 2,4-Dichlorphenylgruppe ist und A eine n-Butoxygruppe ist.

14. Verfahren zur Herstellung einer Verbindung nach Anspruch 3, worin Ph eine 2,4-Dichlorphenylgruppe ist und A eine Isoamyloxygruppe ist.

15. Verfahren zur Herstellung einer Verbindung nach Anspruch 4, worin Ph eine 2,4-Dichlorphenylgruppe ist und A eine n-Hexylaminogruppe ist.

16. Verfahren zur Herstellung einer Verbindung nach Anspruch 4, worin Ph eine 2,4-Dichlorphenylgruppe ist und A eine Benzylaminogruppe ist.

17. Verfahren zur Herstellung einer Verbindung nach Anspruch 4, worin Ph eine 2,4-Dichlorphenylgruppe ist und A eine 2-Hydroxyethylaminogruppe ist.

18. Verfahren zur Herstellung einer Verbindung nach Anspruch 4, worin Ph eine 2,4-Dichlorphenylgruppe ist und A eine N-Methylpiperazinogruppe ist.

19. Verfahren zur Herstellung einer Verbindung nach Anspruch 4, worin Ph eine 2,4-Dichlorphenylgruppe ist und A eine N-Phenylpiperazinogruppe ist.

20. Verfahren zur Herstellung einer antifugalen Zubereitung, dadurch gekennzeichnet, dass man eine antifungal wirksame Menge mindestens einer der Imidazolylpropanolverbindungen und ihrer Säureadditionssalze gemäss Anspruch 1 als Wirkstoffkomponente mit mindestens einem inerten Träger und/ oder mindestens einem inerten Verdünnungsmittel formuliert.

**Revendications pour les Etats contractants: BE CH LI DE FR GB IT NL SE**

1. Dérivé d'imidazolyl-propanol de la formule

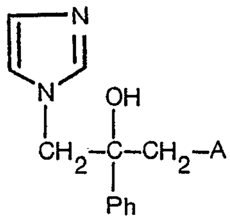

(I)

dans laquelle

Ph désigne un groupe phényle ou un groupe phényle halo-substitué et

A est un groupe —S—$R^1$, où $R^1$ représente un groupe alkyle en $C_1$ à $C_{15}$ ou en $C_{18}$, un groupe phényle, un groupe phényle substitué par au moins un radical alkyle en $C_1$ à $C_8$ ou alcoxy en $C_1$ à $C_8$ ou halogène, un groupe benzyle ou un groupe pyridyle, ou

—O—$R^2$, où $R^2$ représente un atome d'hydrogène, un un groupe alkyle en $C_1$ à $C_{15}$, un groupe alkyle en $C_1$ à $C_8$ substitué par au moins un groupe phényl-thio ou alkyl(en $C_1$ à $C_8$)-thio, un groupe alcényle en $C_3$ à $C_8$ ou un groupe aralkyle en $C_7$ ou $C_8$, éventuellement halo-substitué, ou

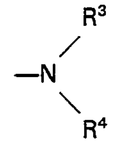

,

où $R^3$ et $R^4$ désignent chacun un atome d'hydrogène, un groupe alkyle en $C_1$ à $C_8$, un groupe cycloalkyle en $C_3$ à $C_6$, un groupe benzyle éventuellement halo-substitué, un groupe alkyle en $C_1$ à $C_8$ substitué par au moins un groupe hydroxyle, morpholino ou alcoxy en $C_1$ à $C_8$, un groupe alcynyle en $C_3$ à $C_6$ ou un groupe furfuryle ou tétrahydrofurfuryle, ou forment avec l'atome d'azote adjacent un groupe morpholino, pipérazino, N-alkyl(en $C_1$ à $C_8$)-pipérazino, N-phénylpipérazino, N-benzyl-pipérazino, pyrrolidino ou pipéridino,

et ses sels d'addition d'un acide.

2. Le composé selon la revendication 1, où A = —S—$R^1$.

3. Le composé selon la revendication 1, où A = —O—$R^2$.

4. Le composé selon la revendication 1, où A =

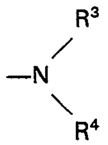

.

5. Le composé selon la revendication 2, où Ph est un groupe phényle halo-substitué et A un groupe alkyl (en $C_1$ à $C_{15}$)-thio.

6. Le composé selon la revendication 2, où Ph est un groupe dichloro-2,4 phényle et A un groupe phényl-thio.

7. Le composé selon la revendication 2, où Ph est un groupe dichloro-2,4 phényle et A un groupe méthyl-3 phényl-thio.

8. Le composé selon la revendication 2, où Ph est un groupe dichloro-2,4 phényle et A un groupe méthoxy-4 phényl-thio.

9. Le composé selon la revendication 2, où Ph est un groupe dichloro-2,4 phényle et A un groupe fluoro-4 phényl-thio.

10. Le composé selon la revendication 2, où Ph est un groupe dichloro-2,4 phényle et A un groupe benzyl-thio.

11. Le composé selon la revendication 5, où Ph est un groupe dichloro-2,4 phényle et A un groupe n-butyl-thio.

12. Le composé selon la revendication 2, où Ph est un groupe dichloro-2,4 phényle et A un groupe pyridyl-4-thio.

13. Le composé selon la revendication 3, où Ph est un groupe dichloro-2,4 phényle et A un groupe n-butoxy.

14. Le composé selon la revendication 3, où Ph est un groupe dichloro-2,4 phényle et A un groupe groupe isoamyloxy.

15. Le composé selon la revendication 4, où Ph est un groupe dichloro-2,4 phényle et A un groupe n-hexyl-amino.

16. Le composé selon la revendication 4, où Ph est un groupe dichloro-2,4 phényle et A un groupe benzylamino.

17. Le composé selon la revendication 4, où Ph est un groupe dichloro-2,4 phényle et A un groupe hydroxy-éthyl-2 amino.

18. Le composé selon la revendication 4, où Ph est un groupe dichloro-2,4 phényle et A un groupe N-méthyl-pipérazino.

19. Le composé selon la revendication 4, où Ph est un groupe dichloro-2,4 phényle et A un groupe N-phényl-pipérazino.

20. Procédé de préparation de dérivés de l'imidazolyl-propanol de la formule I selon la revendication 1 et de leurs sels d'addition d'acides, caractérisé en ce que l'on fait réagir:

a) soit un époxyde de la formule

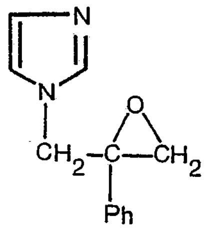

(II)

dans laquelle Ph possède l'une des significations définies dans la revendication 1, avec un réactif de l'une des formules ci-après:

$\overset{\oplus\ominus}{MS}-R^1$ (IIIa) $\overset{\oplus\ominus}{MO}-R^2$ (IIIb) ou 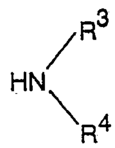 (IIIc) ,

dans lesquelles $R^1$, $R^2$, $R^3$ et $R^4$ possèdent les significations définies dans la revendication 1 et M désigne un atome de métal alcalin,

b) soit un époxyde de la formule

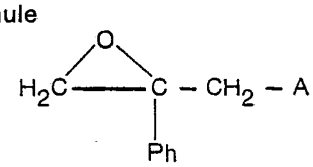

(IV) ,

dans laquelle Ph et A possèdent les significations définies dans la revendication 1, avec un dérivé d'imidazole de la formule

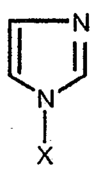

(V)

dans laquelle X désigne un atome de potassium ou de sodium.

21. Composition anti-fongique, contenant une quantité efficace du point de vue anti-fongique d'au moins un dérivé d'imidazolyl-propanol selon la revendication 1 ou sel d'addition d'acide d'un tel dérivé en tant que principe actif dans au moins un véhicule ou diluant inerte.

22. Dérivés de l'imidazolyl-propanol selon la revendication 1 et leurs sels d'addition d'acides, utilisés comme agents antifongiques.

**Revendications pour l'Etat contractant: AT**

1. Procédé de préparation de dérivés d'imidazolyl-propanol de la formule

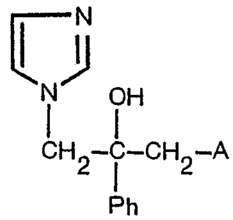

(I)

dans laquelle

Ph désigne un groupe phényle ou un groupe phényle halo-substitué et

A est un groupe —S—$R^1$, où $R^1$ représente un groupe alkyle en $C_1$ à $C_{15}$ ou en $C_{18}$, un groupe phényle, un groupe phényle substitué par au moins un radical alkyle en $C_1$ à $C_8$ ou alcoxy en $C_1$ à $C_8$ ou halogène, un groupe benzyle ou un groupe pyridyle, ou

—O—$R^2$, où $R^2$ représente un atome d'hydrogène, un un groupe alkyle en $C_1$ à $C_{15}$, un groupe alkyle en $C_1$ à $C_8$ substitué par au moins un groupe phényl-thio ou alkyl(en $C_1$ à $C_8$)-thio, un groupe alcényle en $C_3$ à $C_8$ ou un groupe aralkyle en $C_7$ ou $C_8$, éventuellement halo-substitué, ou

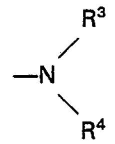
,

où $R^3$ et $R^4$ désignent chacun un atome d'hydrogène, un groupe alkyle en $C_1$ à $C_8$, un groupe cycloalkyle en $C_3$ à $C_6$, un groupe benzyle éventuellement halo-substitué, un groupe alkyle en $C_1$ à $C_8$ substitué par au moins un groupe hydroxyle, morpholino ou alcoxy en $C_1$ à $C_8$, un groupe alcynyle en $C_3$ à $C_6$ ou un groupe furfuryle ou tétrahydrofurfuryle, ou forment avec l'atome d'azote adjacent un groupe morpholino, pipérazino, N-alkyl(en $C_1$ à $C_8$)-pipérazino, N-phényl-pipérazino, N-benzyl-pipérazino, pyrrolidino ou pipéridino,

et de leurs sels d'addition d'un acide,

caractérisé en ce que l'on fait réagir:

a) soit un époxyde de la formule

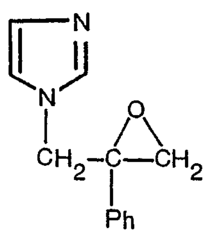

(II)

dans lequelle Ph possède l'une des significations définies précédemment, avec un réactif de l'une des formules ci-après:

$\overset{\oplus\ominus}{MS}-R^1$ (IIIa) $\overset{\oplus\ominus}{MO}-R^2$ (IIIb) ou 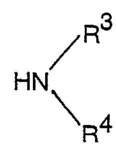 (IIIc) ,

dans lesquelles $R^1$, $R^2$, $R^3$ et $R^4$ possèdent les significations définies précédemment, et M désigne un atom de métal alcalin,

b) soit un époxyde de la formule

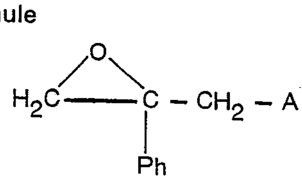

(IV) ,

dans laquelle Ph et A possèdent les significations définies précédemment, avec un dérivé d'imidazole de la formule

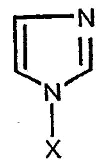

(V)

dans laquelle X désigne un atome de potassium ou de sodium.

2. Procédé pour la préparation d'un composé selon la revendication 1, où A = —S—$R^1$.

3. Procédé pour la préparation d'un composé selon la revendication 1, où A = —O—$R^2$.

4. Procédé pour la préparation d'un composé selon la revendication 1, où A =

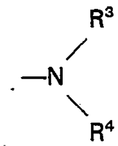

5. Procédé de préparation d'un composé selon la revendication 2, où Ph est un groupe phényle halo-substitué et A un groupe alkyl(en $C_1$ à $C_{15}$)-thio.

6. Procédé de préparation d'un composè selon la revendication 2, où Ph est un groupe dichloro-2,4 phényle et A un groupe phényl-thio.

7. Procédé de préparation d'un composè selon la revendication 2, où Ph est un groupe dichloro-2,4 phényle et A un groupe méthyl-3 phényl-thio.

8. Procédé de préparation d'un composè selon la revendication 2, où Ph est un groupe dichloro-2,4 phényle et A un groupe méthoxy-4 phényl-thio.

9. Procédé de préparation d'un composè selon la revendication 2, où Ph est un groupe dichloro-2,4 phényle et A un groupe fluoro-4 phényl-thio.

10. Procédé de préparation d'un composè selon la revendication 2, où Ph est un groupe dichloro-2,4 phényle et A un groupe benzyl-thio.

11. Procédé de préparation d'un composè selon la revendication 5, où Ph est un groupe dichloro-2,4 phényle et A un groupe n-butyl-thio.

12. Procédé de préparation d'un composè selon la revendication 2, où Ph est un groupe dichloro-2,4 phényle et A un groupe pyridyl-4 thio.

13. Procédé de préparation d'un composè selon la revendication 3, où Ph est un groupe dichloro-2,4 phényle et A un groupe n-butoxy.

14. Procédé de préparation d'un composè selon la revendication 3, où Ph est un groupe dichloro-2,4 phényle et A un groupe isoamyloxy.

15. Procédé de préparation d'un composè selon la revendication 4, où Ph est un groupe dichloro-2,4 phényle et A un groupe n-hexyl-amino.

16. Procédé de préparation d'un composè selon la revendication 4, où Ph est un groupe dichloro-2,4 phényle et A un groupe benzyl-amino.

17. Procédé de préparation d'un composè selon la revendication 4, où Ph est un groupe dichloro-2,4 phényle et A un groupe hydroxy-éthyl-2 amino.

18. Procédé de préparation d'un composè selon la revendication 4, où Ph est un groupe dichloro-2,4 phényle et A un groupe N-méthyl-pipérazino.

19. Procédé de préparation d'un composè selon la revendication 4, où Ph est un groupe dichloro-2,4 phényle et A un groupe N-phényl-pipérazino.

20. Procédé de préparation d'une composition antifongique, consistant à mélanger une quantité efficace du point de vue anti-fongique d'au moins un dérivé d'imidazolyl-propanol selon la revendication 1 ou d'un sel d'addition d'un acide d'un tel dérivé, en tant que principe actif, avec au moins un véhicule ou diluant inerte.